**Europäisches Patentamt**

**European Patent Office**

**Office européen des brevets**

(19)

(11) Veröffentlichungsnummer: **0 664 450 A1**

# (12) EUROPÄISCHE PATENTANMELDUNG

(21) Anmeldenummer: **95100589.1**

(22) Anmeldetag: **18.01.95**

(51) Int. Cl.6: **G01N 33/20**, G01N 33/52, G01N 33/68, C12N 1/00

(30) Priorität: **22.01.94 DE 4401868**

(43) Veröffentlichungstag der Anmeldung:
**26.07.95 Patentblatt 95/30**

(84) Benannte Vertragsstaaten:
**AT BE DE DK ES FR GB GR IE IT NL SE**

(71) Anmelder: **Lobbe Xenex GmbH**
**Stenglingser Weg 4-12**
**D-58642 Iserlohn (DE)**

(72) Erfinder: **Hengstenberg, Wolfgang**
**Universitätsstrasse 150**
**D-44789 Bochum (DE)**
Erfinder: **Kolar, Roman**
**Wörthstrasse 24**
**D-81667 München (DE)**
Erfinder: **Scholz, Marko**
**Hunschneidtstrasse 43**
**D-44789 Bochum (DE)**

(74) Vertreter: **Dr. Fuchs, Dr. Luderschmidt Dr.**
**Mehler, Dipl.-Ing Weiss Patentanwälte**
**Postfach 46 60**
**D-65036 Wiesbaden (DE)**

(54) **Biotest.**

(57) 1. Mikrobielles Verfahren zum Nachweis von Schadstoffen.

2.1. Der bisher zum Nachweis von Schadstoffen eingesetzte äußerst schnell durchführbare Leuchtbakterientest reagiert mit sehr hoher Empfindlichkeit auf Schwermetalle wie Quecksilber und Blei, jedoch nicht auf andere toxikoligisch relevante Schwermetalle wie Cadmium und Chrom. Das neue mikrobielle Verfahren soll in Ergänzung zum Leuchtbakterientest mit hoher Empfindlichkeit und Spezifität auf die ökotoxikologisch relevanten Schwermetalle Cadmium und Chrom ansprechen.

2.2. Als Nachweis für die Gegenwart von Schadstoffen in Proben wird deren Hemmwirkung auf das Phospho-transferasesystem von Bakterien, insbesondere die E.coli-Mutante 1219bgl +, herangezogen. Dieses Testverfahren spricht mit hoher Empfindlichkeit auf die Gegenwart der Schwermetalle Cadmium und Chrom an.

2.3. Das Testverfahren bildet eine Ergänzung zum Leuchtbakterientest und erlaubt es, für den Fall, daß nur eine geringe oder gar keine Hemmung des Phosphotransferase-Systems nachweisbar ist, die Gegenwart von toxikologisch relevanten Konzentrationen an Cadmium und Chrom in der Probe äußerst schnell auszuschließen.

EP 0 664 450 A1

Die Erfindung betrifft ein mikrobielles Verfahren zum Nachweis von Schadstoffen, insbesondere von Schwermetallen mittels Bestimmung der Wirkung der in einer Probe enthaltenen Schadstoffe auf den Stoffwechsel von Mikroorganismen.

Im Stand der Technik sind eine Vielzahl von Biotests zum Schadstoffnachweis bekannt. Die Anforderungen an solche Biotests sind sehr vielfältig, wie z.B. die Standardisierbarkeit, andauernde Verfügbarkeit, Reproduzierbarkeit, hohe Empfindlichkeit bzw. niedrige Nachweisgrenze, große Schnelligkeit und problemlose Handhabung, kostengünstige Durchführung und nicht zuletzt eine zuverlässige Korrelation gemessener Wirkungen zu biologischen Prozessen. Die Summe dieser Anforderungen bringt es mit sich, daß auch Probleme bezüglich der Aussagekraft der gemessenen Ergebnisse auftreten können. So schließen sich z.B. die Anforderungen einer großen Schnelligkeit bei geringer Nachweisgrenze und eine hohe Reproduzierbarkeit gegenseitig meistens aus. Darüber hinaus sind gewisse Umweltchemikalien für den einen Organismus toxisch, für den anderen jedoch nicht. Im Bereich der Abwasserreinigung existieren daher auch lt. DIN-Norm verschiedene Biotests mit Organismen verschiedener Organisationshöhe. Die verwendeten Testorganismen sollen dabei jeweils die wichtigsten Organismen in Oberflächengewässern repräsentieren:

Tabelle 1

| Biotest | Testorganismus | Untersuchungsparameter |
|---|---|---|
| Fischtest DIN 38412, Teil 31 | Goldorfe<br><br>*Leuciscus idus* | $LC_0$: max. Konzentration, die nach 48 h Inkubation zu 0% Mortalität führt |
| Daphnientest DIN 38412, Teil 30 | Wasserfloh<br><br>*Daphnia magna* | $G_D$: kleinste Verdünungsstufe, bei der 90% der Testtiere ihre Schwimmfähigkeit behalten |
| Algentest DIN 38412, Teil 33 | Grünalge<br><br>*Scenedesmus subspicatus* | $H_F$: Hemmwirkung der Chlorophyll-Fluoreszenz auf die Biomassenproduktion in % |
| Leuchtbakterientest DIN 38412, Teil 34 | Bakterien<br><br>*Vibrio fischeri* | $EC_{20}$: max. Konzentration, die nach 20 min Inkubation zu 20% iger Hemmung der Lichtemission führt |

Diese Biotests sind jedoch nicht als Alternativtests aufzufassen. Da die einzelnen Organismen sowohl mit unterschiedlicher Empfindlichkeit als auch unterschiedlicher Spezifität auf einen jeweiligen Schadstoff bzw. eine Kombination von Schadstoffen ansprechen, lassen sich aus diesen Tests nur in Kombination miteinander vernünftige Aussagen über ein in einer Probe jeweils vorhandenes Schadstoffpotential bzw. die allgemeine Schädigungskapazität machen, bevor dann einzelne Schadstoffe der Probe individuell quantitativ bestimmt werden.

In Tabelle 2 ist beispielhaft ein ökotoxilogisches Prüfprogramm mit unterschiedlichen biologischen Prüfparametern wiedergegeben, wie es vom Bundesumweltamt vorgeschlagen wurde. Die einzelnen Biotests zeichnen sich durch unterschiedlichen apparativen als auch zeitlichen Aufwand aus. So ist die Wirkung von Schadstoffen, insbesondere im Goldorfentest, im Daphnientest und im Grünalgentest auf die Langzeitwirkung des gesamten Organismus auf die einwirkenden Schadstoffe gerichtet. Für solche Testmethoden benötigt man daher einen relativ langen Zeitaufwand. Darüber hinaus erfordern z.B. Fischtests eine relativ aufwendige und teure Haltung der Testorganismen.

**Tabelle 2**: Okotoxikologische Prüfungen der Grundstufe und Stufe 1

| Prüfmethode | Prüfparameter | Prüforganismus |
|---|---|---|
| **Grundstufe** | | |
| Akute Toxizität an einer Fischart | Tödliche Wirkung des Stoffes nach einmaliger Applikation während 24 bis 48 Stunden | Zebrabärbling Brachdydanio rerio Sekundärkonsument |
| Akute Toxizität an einer Wasserflohart | Hemmung der Schwimmfähigkeit nach einmalaiger Applikation während 24 bis 48 Stunden | Großer Wasserfloh Daphnia magna Primärkonsument |

## Stufe 1

| | | |
|---|---|---|
| Algentoxizität | Hemmung der Zellvermehrung (Wachstum) nach einmaliger Applikation während 72 Stunden | Grünalge Scenedesmus subspicatus Primärproduzent |
| Langfristige Daphnientoxizität | Ermittlung der No-Observed-Effect Concentration und der Schwellenkonzentration in Bezug auf Beeinträchtigung der Reproduktionsleistung und tödliche Wirkung bei wiederholter (semistatisches) oder ständiger (Durchfluß-System) Applikation während mindestens 21 Tagen | Großer Wasserfloh Daphnia magna |
| Langfristige Fischtoxizität | Ermittlung der No-Observed-Effect-Concentration und der Schwellenkonzentration in Bezug auf tödliche und subletale Wirkungen bei wiederholter (semistatisches) oder ständiger (Durchfluß-System) Applikation während 14 bis 28 Tagen | Zebrabärbling Brachydanio rerio |
| Pflanzentoxizität | Hemmung des Wachstums von Saatgut (Verringerung der Biomasse) während 14 Tagen nach einmaliger Applikation | Hafer Avena sativa Rübe Brassica rapa Primärproduzent |
| Regenwurmtoxizität | Tödliche Wirkung des Stoffes während 14 Tagen nach einmaliger Application | Regenwurm Eisenia foetida Sekundärdestruent |

## Stufe 2

Individuelles stoffspezifisches Prüfprogramm

Wesentlich schnellere Ergebnisse mit geringerem Aufwand lassen sich mit den sogenannten "akuten Biotests" unter Einsatz von Mikroorganismen erzielen. Hierbei wird die Wirkung auf einen Schadstoff nur über ein meßbaren Parameter eines Organismus bestimmt, welcher mit der "effektiven Konzentration" (EC-Wert) als toxikologischer Kenngröße korreliert sein kann.

So ist beispielsweise der in der DE-PS 28 41 896 beschriebene Leuchtbakterientest ein Verfahren, bei dem zum Nachweis einer toxischen Substanz oder eines toxischen Zustandes die Änderung der von einem

4

Biolumineszenzorganismus ausgehenden Lichtemission als Maß der Einwirkung der toxischen Substanz bzw. des toxischen Zustandes auf den Stoffwechsel des Biolumineszenzorganismus bestimmt wird. Ein solcher mikrobieller Test ist im Vergleich zu einem biologischen Test mit Eukaryonten äußerst schnell durchführbar (wenige Minuten), hat aber den Nachteil, da hierbei ein Prokaryont eingesetzt wird, daß eine Korrelation der beobachteten Schadstoffwirkung zu deren Wirkung auf Eukaryonten nicht ohne weiteres möglich ist.

Es zeigte sich jedoch, daß die im Leuchtbakterientest eingesetzten Mikroorganismen äußerst empfindlich und spezifisch auf die toxischen Schwermetalle Quecksilber und Blei ansprechen. Dies bietet nun wieder den Vorteil, daß in Vorversuchen äußerst rasch geklärt werden kann, ob in einer Probe gerade diese Schwermetalle als Schadstoffe enthalten sein können. Bei negativem Ergebnis im Leuchbakerientest kann dann nämlich von vornherein, innerhalb von Minuten, das Vorhandensein von Quecksilber und Blei in toxikologischen Mengen ausgeschlossen werden.

Nun ließen sich aber mit dem Leuchtbakterientest, dem bisher einzigen bekannten Bakterientest, zwei weitere äußerst wichtige toxische Schwermetalle, nämlich Cadmium und Chrom nicht erfassen bzw. der Test spricht nicht mit genügender Empfindlichkeit an. Letzteres trifft insbesondere auf Cadmium zu, welches als $CdCl_2$ an Nr. 22 in der vom Bundesgesundheitsamt herausgegebenen Liste der Stoffe des "25-Stoffe-Programms" enthalten ist.

Aufgabe der Erfindung ist daher, einen weiteren Bakerientest zur Verfügung zu stellen, mit welchem die Gegenwart der toxischen Schwermetalle Cadmium und Chrom in einer Probe mit einer so großen Empfindlichkeit bzw. Spezifität erfaßt werden kann, daß bei Nichtansprechen des Testsystems das Vorkommen von Cadmium und Chrom in der Probe in toxikologischen Konzentrationen ausgeschlossen werden kann.

Die Aufgabe wird dadurch gelöst, daß als Nachweis für die Gegenwart von Schadstoffen in Proben deren Hemmwirkung auf das Phototransferase-System (PTS) von Bakterien herangezogen wird. Vorteilhafte Ausführungsformen werden in den Unteransprüchen wiedergegeben.

Das bakterielle Phosphotransferase-System ist ein Multienzymkomplex und bildet einen essentiellen Bestandteil zur Bereitstellung von Energie in Bakterien. Es besteht aus den Bausteinen Enzym I (EI) und HPr (heat stable protein/histidin protein) welche im Zytoplasma lokalisiert, unspezifisch gegenüber Kohlenhydraten sind und konstitutiv exprimiert werden. Weitere Bausteine bilden die kohlenhydratspezifischen Enzyme II (EII), welche induzierbar sind und eine hetorogene Gruppe darstellen mit einer variablen Anordnung ihrer funktionellen Domänen. Für E.coli lassen sich z.B. folgende Anordnungen angeben:

1. Das Mannitol-PTS besteht aus einem einzelnen, membrangebundenen Protein, das aus den drei Domänen A, B und C aufgebaut ist: IIABC.

2. Das Glukose-PTS besteht aus zwei oder mehr Proteinen, von denen eines in gelöster Form mit einer Domäne vorliegt und das andere membrangebunden mit zwei Domänen: IIA, IICB.

3. Im Mannose-PTS sind IIA und IIB als singuläre cytoplasmalösliche Polypeptide fusioniert, wobei die Zuckertranslokation über die Membran mittels zweier integraler Membranproteine IIC und IID erfolgt.

In jedem Fall erfolgt der Phosphorylgruppentransfer ausgehend von Phosphoenolpyruvat (PEP) über EI, HPr, IIA und IIB. Die IIC-Domäne stellt den integralen Membrananteil des EII dar, der den Transportkanal ausbildet und die zuckerspezifische Bindungsstelle aufweist.

Neben den oben beschriebenen Phosphotransferase-Systemen für Glucose, Mannose und Mannitol treten bei anderen Mikroorganismenspezies auch weitere, weniger häufig Organisationsformen auf.

Das EI des PTS wird an der N3-Position eines Histidyl-Rests durch PEP phosphoryliert. Die Phosphorylierung erfolgt in der dimeren Form des Proteins, wobei jedes Monomer eine Phosphorylgruppe trägt.

Die Phosphorylierung des HPr durch phosphoryliertes EI erfolgt an der N1-Position eines Histidylrests (His 15 in E.coli). Ausgehend von phosphoryliertem HPr erfolgt die weitere Übertragung der Phosphorylgruppe dann auf kohlenhydratspezifische Domänen, die als eine sehr hetrogene Gruppe vorkommen. Die meisten EII weisen die drei Domänen IIA, IIB und IIC auf. IIA und IIB stellen jeweils die hydrophilen und IIC (und ggfs. IID) die lipophilen Domänen dar. Während den IIA- und IIB-Domänen essentielle Bedeutung beim Transfer der Phosphorylgruppen zukommt, stellen die IIC- bzw. IID-Domänen die Kohlenhydraterkennungsstelle sowie den Transmembrankanal dar. Vom phosphorylierten HPr wird die Phosphorylgruppe zunächst auf einen Histidylrest der IIA-Domäne übertragen. Weniger Informationen liegen bisher von den hydrophilen IIB-Domänen vor, die an dem Phosphorylgruppentransfer über Cysteylgruppen beteiligt sind. Die IIC-Domänen durchspannen als Helices mehrfach die Zellmembran. Die Ausbildung des Transmembrankanals geschieht möglicherweise über Oligomerisierung von mehreren IIC's der entsprechenden E II-Proteine, um eine ausreichende Anzahl von amphipatischen Alpha-Helices zur Verfügung zu stellen.

Da das Phototransferase-System für die Bakterienzelle ein essentielles Transportsystem zur Aufnahme extrazellulärer Kohlenhydrate ist, sollte sich eine eventuelle Hemmung bzw. Beeinträchtigung der daran

beteiligten Enzyme in einer Verringerung des Kohlenhydrattransports bemerkbar machen, d.h., das PTS bildet eine potentielle Möglichkeit zur Detektion von Schadstoffen in Mikroorganismen. Es ist bekannt, daß Schadstoffe wie beispielsweise organische Lösungsmittel oder Detergenzien Enzymaktivitäten hemmen. Dies gilt insbesondere auch für Schwermetalle, welche als starke Enzyminhibitoren gelten. Erfindungsgemäß wurde nun anstelle eines Kohlenhydrats ein Substratanaloges zum Transport durch die Zellmembran eingesetzt, welches so beschaffen ist, daß es in phosphorylierter Form nach dem Zelltransport von einem Enzym in der Bakterienzelle hydrolisiert werden kann unter Freisetzung einer analytisch erfaßbaren Substanz. Wird z.B. 2-Nitrophenyl-$\beta$-D-Glucosid als Substratanaloges eingesetzt, dann wird dieses vom PTS unter Phosphorylierung in eine Bakterienzelle eingeschleust wo das ONP-Glc-6-P von der induzierbaren 6-Phospho-$\beta$-Glucosidase B zu Glc-6-P und 2-Nitrophenol hydrolytisch gespalten wird. Das freigesetzte 2-Nitrophenol kann dann nach einer definierten Zeitspanne mittels photometrischer Bestimmung als Phenolat-Anion quantitativ gemessen werden. Darüber hinaus steht mit der E.coli-Mutante 219bgl + ein Bakterium zur Vefügung, welches sich durch erhöhte $\beta$- glycosidische Aktivität auszeichnet, dabei aber nicht gentechnisch verändert noch patogen ist, was eine wesentliche Voraussetzung für gefahrloses Arbeiten mit Bakterien darstellt.

Die Durchführung eines PTS-Hemmtests wird nun im folgenden beschrieben:

Die nachstehend wiedergegebenen optimalen Reaktionsparameter beziehen sich auf die Verwendung der E.coli-Mutante 1219bgl + mit der Hinterlegungsnummer DSM 8779. Bei Verwendung anderer das PTS aufweisenden Bakterienstämme sind zur Optimierung ggfs. abweichende Werte für die Reäktionsparameter einzustellen, welche dann mittels Vorversuchen ermittelt werden müßten.

In den Figuren 1 bis 4 werden die funktionellen Zusammenhänge einiger Parameter wiedergegeben. Es zeigen:

Figur 1: Die Abhängigkeit der Hydrolyserate (in %-Restaktivität) von ONP-Glucose in E.coli von der Konzentration der Rinderserumalbumin-Lösung, in welcher die Bakterien gewaschen wurden.

Figur 2: Die Abhängigkeit des Umsatzes von ONP-Glykosid in Abhängigkeit vom pH-Wert.

Figur 3: Die Temperaturabhängigkeit des Umsatzes von ONP-Glykosid.

Figur 4: Die Abhängigkeit der ONP-Glykosidspaltung in Abhängigkeit von der Substratkonzentration.

Vor dem Einsatz der Bakterien müssen diese auf ein intaktes PTS untersucht werden. Hierzu werden Zellen des entsprechenden Bakterienstammes auf UMB-Selektivplatten ausgestrichen und bei 30° C bebrütet (UMB = 4-Methylumbelliferon als Fluoreszenzindikator). Die Herstellung der UMB-Selektivplatten erfolgt mittels 15 g Agar, 25 ml Salicin (20%, w/v), welches das $\beta$-Glucosid-PTS induziert und als Testsubstrat für $\beta$-Glykosidasen dient, und beide werden ad 1000 ml TBY-Medium aufgefüllt. Das TBY-Medium setzt sich aus 10 g Trypton, 5 g Hefeextrakt, 5 g NaCl welches ad 1000 ml Aqua dest. aufgefüllt wurde zusammen. Falls Kohlenhydrate zur Anzucht der Bakterien erforderlich sind, werden sie separat autoklaviert und nach dem Abkühlen unter sterilen Bedingungen auf eine Endkonzentration von 0,5% (w/v) gebracht. Auf die Agar-Platten werden dann 50 $\mu$l UMB-$\beta$-D-Glucosid (1 $\mu$g/ml DMSO) ausgestrichen.

UMB-Glucose dient als Substratanalogon zu Salicin. Salicin ist ein Testsubstrat für $\beta$-Glykosidasen und wird durch das PTS in die Zelle transportiert und phosphoryliert. Analog hierzu wird dann die UMB-Glucose zu UMB-Glc-6-P phosphoryliert und danach durch die induzierbare 6-Phospho-$\beta$-Glukosidase B zu Glucose-6-P und UMB hydrolysiert. Das freigesetzte UMB dient infolge seiner fluoreszierenden Eigenschaft nach Anregung mit Licht bei einer Wellenlänge von 365 nm als Fluoreszenzindikator für ein intaktes PTS.

Um Testbakterien bei Bedarf schnell und in ausreichender Menge zur Verfügung zu haben, muß zuvor ein Vorrat an konservierten Zellen angelegt werden. Hierzu wird das oben beschriebene TBY-Medium mit ein Zehntel Volumen einer Kolonie von Bakterien des entsprechenden Stammes beimpft und bei 30° C bebrütet (z.B. in einem Rundschüttler bei 180 U/min). Die Induktion des bgl-PTS bei E.coli 1219 erfolgt durch unmittelbare Zugabe von Salicin in das Medium auf eine Endkonzentration von 0,5% (w/v).

Das Wachstum der Zellen wird durch Messen der OD (optischen Dichte) bei 578 nm kontrolliert, die Induktion des PTS durch Messen der OD bei 405 nm nach Induktion mit ONP-$\beta$-D-Glucosid (stellvertretend für 2-Nitrophenyl-$\beta$-D-Glucosid (ONP-Glucosid) kann auch 4-Nitrophenyl-$\beta$-D-Glucosid eingesetzt werden, ohne in den folgenden Ausführungen ausdrücklich erwähnt zu werden). Haben die Zellen in der Dispersion eine $OD_{578}$ von 2,0 erreicht, werden die Zellen durch 10 min Zentrifigation bei 17 000 x g (bei großen Volumina nach 20 min mit 11 000 x g) geerntet.

Der pH-Wert wird sodann durch Zugabe von 50 mM eines physiologisch verwendbaren Puffers auf pH 6,5 eingestellt, wobei stets der Puffer eingesetzt wird, in dem auch der PTS-Test durchgeführt werden soll. Als Puffer kommen vorzugsweise in Frage:

HEPES-Puffer (50 mM) mit NaOH auf pH 6,5-7,5 eingestellt.

Bis/Tris-Puffer (50mM) mit HCl auf pH 6,5 eingestellt.

Tris/HCl-Puffer (50mM) mit HCl auf pH 6,5 eingestellt.

Phosphatpuffer: Lösung A: $Na_2HPO_4$ (50 mM, 100 mM); Lösung B: $KH_2PO_4$ (50 mM, 100 mM). Die Lösung A wird vorgegeben, mit Lösung B (gleiche Konzentration) wird auf den entsprechenden pH-Wert eingestellt.

Insbesondere bevorzugt sind HEPES-Puffer und Phosphatpuffer.

Die Konservierung der Bakterienkulturen erfolgt vorzugsweise durch Einfrieren. Zu diesem Zweck müssen die wie oben beschrieben angezüchteten Bakterienkulturen einer Vorbehandlung unterzogen werden. Da aufgrund des Zellwandaufbaus Gram-negativer Bakterien mit einfachem Mureinsacculus die Zellen von E.coli nicht ohne weiteres eingefroren werden können, weil die beim Abkühlen unter den Gefrierpunkt entstehenden Eiskristalle die Zellwand zerstören würden, müßten die Bakterien beim Wiederauftauen lysieren. Dies läßt sich an E.coli-Kulturen anhand der geringen ONP-Glucose-Umsatzraten der ohne Vorbehandlung aufgetauten Bakterienzellen zeigen. Die verbleibende ONP-Bildung der zuvor tiefgefroreren Bakterien beträgt dann lediglich nur noch 16% bezogen auf den Umsatz der Zellen vor dem Konservierungsverfahren. Es zeigte sich, daß die Nativität der wiederaufgetauten Bakterienzellen von dem Waschprozeß vor dem Einfrieren abhängt. Es zeigte sich ferner, daß beim Waschen mit BSA-Lösungen (Rinderserumalbumin) die Zunahme der Restaktivität im gemessenen Bereich proportional mit der BSA-Konzentration der Lösung anstieg (unter Restaktivität wird der Quotient der Substratumsätze nach und vor dem Einfrieren verstanden). Um dies zu demonstrieren wurden Zellen von E.coli zunächst in ein zwanzigstel Volumen verschiedener wässriger Lösungen (Tabelle 3) gewaschen und bei - 20° C eingefroren. Die Bewertung erfolgte, indem die Quotienten der Hydrolyse der ONP-Glucose der Bakterien mit Hilfe des PTS-Tests nach dem Auftauen und vor dem Einfrieren gebildet wurden. Hierdurch ließen sich Aussagen über die Überlebensrate der Bakterien in % Restaktivität machen. In Tabelle 3 sind die prozentualen Restaktivitäten des Umsatzes der ONP-Glucose wiedergegeben, der von den Bakterien erreicht wurde.

Tabelle 3

| Testlösung | Glycerin (88 %) | BSA (1 %) | BSA (10 %) | Glycerin (44%) MgCl₂ (50 mM) |
|---|---|---|---|---|
| Restaktivität | 40,9 % | 26,6 % | **85 %** | 55 % |
| Testlösung | MgCl₂ (50 mM) NaCl (20 mM) | BSA (0,5 %) Glycerin (44 %) | BSA (0,5 %) MgCl₂ (50 mM) | Zellpellet ohne Zusatz |
| Restaktivität | 15,6 % | 51,3 % | 14,3 % | 16 % |

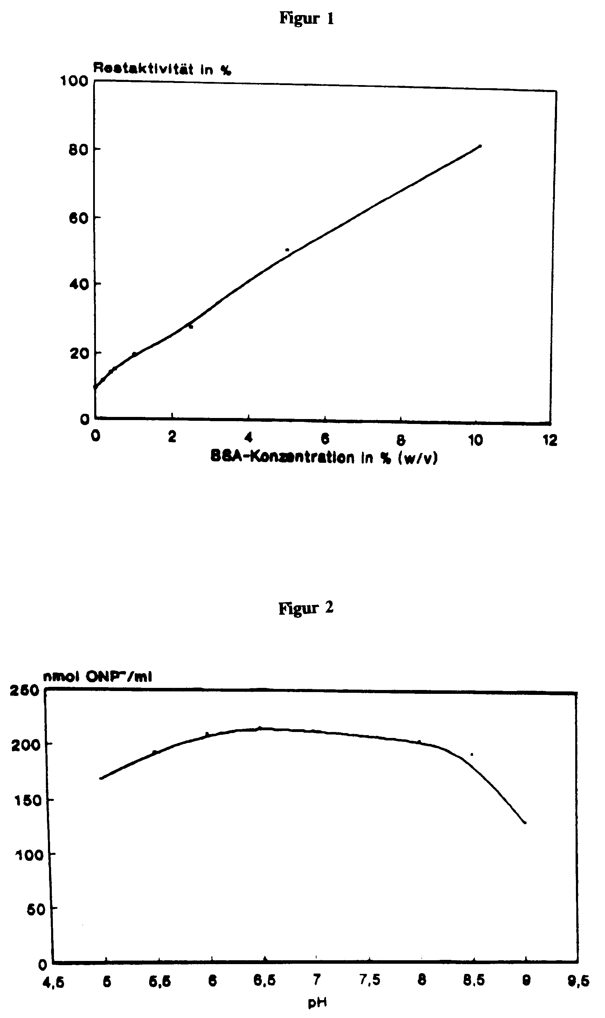

Wie aus Tabelle 3 ersichtlich, wird eine hohe Restaktivität durch Verwenden von BSA-Lösungen in hohen Konzentrationen erreicht. Weiterhin läßt sich entnehmen, daß andere Zusätze zur Konservierung nicht erforderlich sind. Es läßt sich deutlich erkennen, daß im gemessenen Konzentrationsbereich die Hydrolyse der ONP-Glucose von E.coli proportional zur Konzentration der BSA-Lösung ist, in welcher die Bakterien zuvor gewaschen wurden (Fig. 1).

Es ließ sich weiterhin zeigen, daß die Restaktivität der Bakterien nach dem Wiederauftauen unabhängig von der eingesetzten Einfriermethode ist. Hierzu wurden die in 10%-iger BSA-Lösung gewaschenen Mikroorganismen zum Einfrieren bei - 20° C und - 80° C ins Tiefkühlfach und zum Schockgefrieren für 30 Sekunden in ein gemischtes Trockeneis/Aceton-Bad (- 78° C) bzw. in flüssigen Stickstoff (- 196°C) getaucht und dann bei - 20° C bzw. - 80° C gelagert. Die beschriebenen Einfrierverfahren zeigten keine Unterschiede bezüglich der prozentualen Restaktivität

Erfindungsgemäß werden deshalb die Zellen der E.coli-Mutante 1219 bgl + zur Konservierung nach ihrer Ernte in ein Zwanzigstel Volumen BSA-Lösung (10%) gewaschen, portioniert und durch Einfrieren bei - 20° C gelagert, so daß sie in variablen Mengen für die nachfolgenden Tests zur Verfügung stehen. Die durchschnittlichen Restaktivitäten betragen nach dem hier dargestellten Verfahren ca. 85% (Tabelle 2); dieser Wert ließ sich auch nach 6 Monaten Lagerung bei - 20° C erzielen.

Für den Einsatz der bei - 20° C konservierten E.coli-Kulturen müssen diese zunächst reaktiviert werden, indem sie über einen Zeitraum von 10 Minuten bei Raumtemperatur aufgetaut werden.

Für den sich anschließenden Hemmtest müssen die Mikroorganismen vor dem eigentlichen Start der Reaktion mit der Testlösung (ONP-Glucose) in einem geeigneten Puffersystem inkubiert werden. Während der eigentlichen Reaktionszeit sollte die Fähigkeit der Zellen, ONP-Glykoside zu hydrolysieren, nicht abnehmen. Es zeigte sich, daß für E.coli eine Inkubationszeit von 10-15 Minuten bei einer Temperatur von 37° C die besten Ergebnisse liefert. Bei der Mikroorganismenspezies findet während dieser Zeitspanne eine Temperaturadaption statt, nach der ein um etwa 13% erhöhter Umsatz von ONP-Glucose zu verzeichnen ist, der danach über einen Zeitraum von ca. 60 Minuten konstant bleibt, was durch Vorversuche nachgewiesen werden konnte. Dies ist insbesondere vorteilhaft, da die Fähigkeit der Zellen, ONP-Glykoside zu hydrolysieren, über den Reaktionszeitraum nicht abnehmen soll. Ohne eine Klärung dieses Sachverhaltes wäre nämlich nicht eindeutig zu ersehen, ob eine reduzierte ONP-Bildung durch verbrauchte Energiereserven oder durch die Inkubation mit den Schadstoffen hervorgerufen wird. Verfolgt man die ONP-Glykosidhydrolyse bei 37° C ohne Vorinkubation, so wird man finden, daß erst nach etwa 15 Minuten die maximale ONP-Glucose-Spaltung von etwa 194 nmol/ml erreicht wird, was einer Zunahme um 12,9% entspricht. Zwischen 15 Minuten und 45 Minuten bleibt die Umsatzrate nun konstant und nimmt dann bis etwa 90 Minuten nach Reaktionsstart um ca. 13% ab.

Um für den sich anschließenden PTS-Hemmtest genaue und reproduzierbare Daten zu erzielen, müssen die Reaktionsparameter optimal aufeinander abgestimmt sein. Da der Substratumsatz proportional zur Reaktionszeit und zur eingesetzten Bakterienmenge ist, wurde für die Kombination beider Versuchsparameter ein Kompromiß erzielt, der eine maßvolle Umsatzrate in kurzer Zeit ermöglicht. Für ein Volumen von 500 $\mu$l bei 37° C beträgt die Reaktionszeit des Inkubationsansatzes bei einer $OD_{578}$ von 3,0 für die Zellsuspension erfindungsgemäß vorzugsweise 5 Minuten. Die mittels einer Neubauer-Zählkammer ermittelte Zellzahl für die E.coli-Mutante 1219 bgl + belief sich dann auf $2,2 \times 10^{10}$ Zellen/ml.

Verfolgt man den ONP-Glykosidumsatz in Abhängigkeit vom pH-Wert des Puffersystems in einem Bereich zwischen pH 5,0 bis pH 9,0, dann erkennt man, daß der Umsatz für E.coli innerhalb eines breiten pH-Bereichs zwischen pH 5,5 und pH 8,5 relativ konstant bleibt, wobei bei einem pH-Wert über 8,5 die ONP-Glukose-Spaltungsrate rasch abnimmt (Fig. 2). Mit E.coli 1219 steht daher ein Bakterienstamm zur Verfügung, der in einem breiten pH-Bereich einsetzbar ist und zudem noch eine geringe Reaktionszeit (10-15 minütige Vorinkubation und 5 minütige Reaktionsdauer) benötigt. Für den erfindungsgemäßen PTS-Hemmtest wird vorzugsweise ein pH-Wert von 6,5 eingestellt.

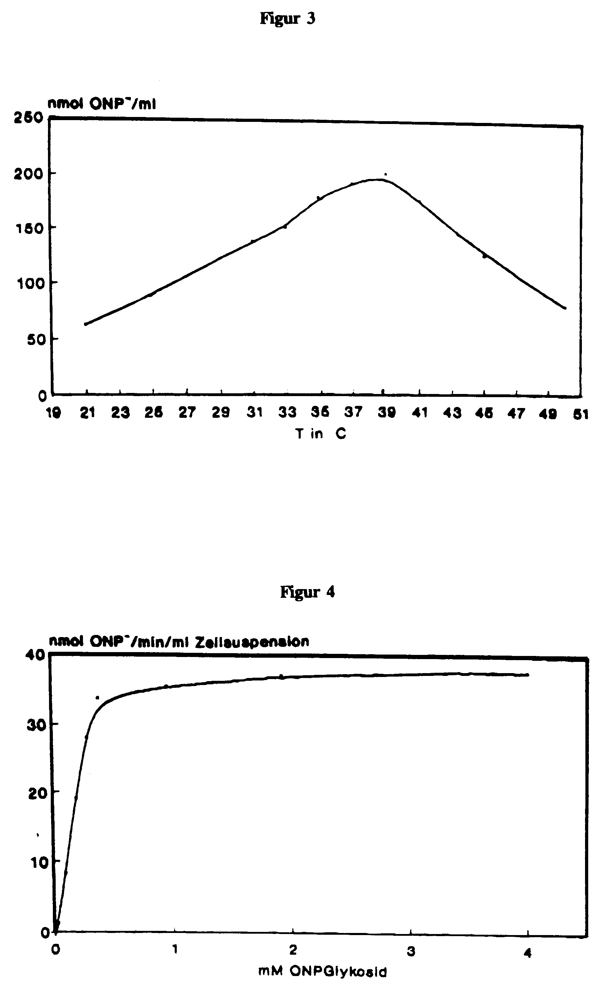

Verfolgt man die Temperaturabhängigkeit der ONP-Glykosidhydrolyse zwischen 21° C und 50° C so erkennt man, daß bei ca. 39° C ein Maximum anzutreffen ist und bei weiterer Temperaturerhöhung die Hydrolyserate schnell absinkt (Fig. 3). Da die ONP-Glykosidhydrolyserate bei der im Labor für physiologische Versuche üblicherweise verwendeten Temperatur von 37° C nur unwesentlich geringer als beim Maximum von 39° C ist, wird für den erfindungsgemäßen Hemmtest eine Temperatur von 37° C gewählt.

Bei Betrachtung der ONP-Glykosid-Spaltung in Abhängigkeit der Substratkonzentration zeigt sich, daß eine Sättigungskinetik ohne Substrathemmung vorliegt (Fig. 4). Mittels des Lineweaver-Burk-Verfahrens wurde eine maximale Reaktionsgeschwindigkeit bei Einsatz von 42 nmol/min/ml ermittelt und ein $K_m$ Wert von 0,21 mM. Da bei weiterer Erhöhung der Substratkonzentration kein weiterer Geschwindigkeitszuwachs zu verzeichnen war, erfolgt die Dosierung des einzusetzenden Substrats erfindungsgemäß nach einer Kosten-Nutzenerwägung zu 2 mM. Bei Durchführung des erfindungsgemäßen PTS-Hemmtests zeigte sich auch, daß die Empfindlichkeit der Tests deutlich von der Art des eingesetzten Puffers abhängt. Insbesondere fiel auf, daß Phosphatpuffer auf die ONP-Glykosidhydrolyse deutlich stimulierend wirkt, während andererseits bei Durchführung des Hemmtests mit Schwermetallen als Schadstoffe die stärksten Hemmeffekte bei Einsatz von HEPES-Puffer zu verzeichnen waren. Hierbei ist jedoch zu berücksichtigen, daß ein Phosphatpuffer in Gegenwart von Schwermetallsalzen wegen dabei evtl. auftretender Löslichkeitsprobleme weniger gut geeignet ist und daher beim Test auf Schwermetalle dem HEPES-Puffer der Vorzug zu geben ist.

Der erfindungsgemäße PTS-Hemmtest wird also wie folgt durchgeführt:

Der Test erfolgt mit einem Reaktionsvolumen von insgesamt 500 $\mu$l in Eppendorf-Cups, wobei die $OD_{578}$ der Bakteriensuspension 3,0 beträgt

Die bei - 20° C konservierten E.coli-Kulturen der Mutante 1219 bgl + werden zur Reaktivierung zunächst über einen Zeitraum von 10 min bei Raumtemperatur aufgetaut, darauf in 250 $\mu$l 50 mM Puffer suspendiert und mit der entsprechenden Testlösung und Aqua dest. auf ein Volumen von 480 $\mu$l gebracht. Diese Suspension wird sodann über einen Zeitraum von 10-15 Minuten bei 37° C vorinkubiert. Danach wird durch Zugabe von 20 $\mu$l ONP-Glucose (50 mM) (entspricht einer Endkonzentration im Reaktionsansatz von 2 mM) die Reaktion gestartet und das Reaktionsgemisch über einen Zeitraum von 5 Minuten bei 37° C inkubiert. Nach Ende dieser Rekationszeit wird durch Zugabe von 1 ml $Na_2CO_3$ (0,5 M) die Reaktion beendet. Nach 2 minütiger Zentrifugation bei 17 000 x g werden die Substratumsätze der getesteten Bakteriensuspensionen photometrisch bei 405 nm bestimmt.

Die Auswertung des PTS-Tests mit ONP-Glykosiden erfolgt mittels der toxikologischen Kenngröße der "effektiven Konzentration" (EC-Wert). Hierunter wird diejenige Konzentration an zugesetztem Schadstoff verstanden , bei der ein meßbarer Parameter eines Organismus innerhalb eines frei definierten Zeitraums um einen bestimmten Betrag gehemmt wird. Durch einen angefügten Index wird die prozentuale Beeinträchtigung des zu messenden Parameters angegeben.

Die Umformung der ermittelten prozentualen Hemmwerte zu EC-Werten erfolgt mit Hilfe des Gammaverfahrens (Johnson, F.H., Eyring, H. and Stover, B.J., 1974; The theory of rate processes in biology and medicine, John Wiley & Sons, New York: 1-385). Mit Hilfe der im PTS-Hemmtest jeweils ermittelten $OD_{405}$ wird der Quotient aus der ONP-Glykosid-Spaltungsabnahme und der verbleibenden ONP-Glykosidspaltung gebildet.

$$\text{Gamma} = \frac{D\ OD_{405}}{OD_{405} - D\ OD_{405}}$$

$OD_{405}$: Substratumsatz ohne Schadstofflösung
$DOD_{405}$: Abnahme des Substratumsatzes nach Inkubation mit der Schadstofflösung
Die hieraus erhaltenen Gamma-Werte werden gegen die eingesetzte Schadstoffkonzentration in doppelt-logarithmischer Darstellung aufgetragen. Mit Hilfe einer anzulegenden Regressionsgeraden lassen sich dann die EC-Werte bestimmen, wobei sich der $EC_{10}$-Wert aus dem Schnittpunkt der Geraden mit der Parallelen zur Abzissenachse in einem Abstand von 0,111 und der $EC_{50}$-Wert in einem Abstand von 1,0 ergibt.

Der erfindungsgemäße PTS-Hemmtest wird nun anhand verschiedener Schadstoffe in den folgenden Beispielen näher erläutert.

Beispiel 1:

PTS-Hemmtest mit verschiedenen Schwermetallen

Der Test wurde mit dem oben beschriebenen Ansatz in HEPES-Puffer bei pH 6,5 durchgeführt. Es zeigte sich, daß z.B. für die Hemmung bei Einsatz von $Hg^{2+}$ die Empfindlichkeit des Tests bei Verwendung eines HEPES-Puffers um den Faktor 50 empfindlicher war als bei Verwendung eines Phosphatpuffers. Bei Einsatz der anderen Puffer traten hemmende Effekte der Metallionen erst bei so hohen Konzentrationen auf, daß es zu Niederschlagsbildung im Reaktionsansatz kam (z.B. $Cd^{2+}$ ab ca. 500 ppm entsprechend 4,5 mM). Um eine möglichst große Empfindlichkeit des Testsystems zu gewährleisten, wurden daher die Untersuchungen im PTS-Hemmtest auf Schwermetalle in HEPES-Puffer durchgeführt.

In den eingesetzten Testlösungen wurden verschiedene Schadstoffkonzentrationen von Cd-, Cr-, Cu-, Hg-, Ni- und Pb-Salzen (durchwegs als Chloride) eingesetzt, bei welchen die Hemmung proportional zur eingesetzten Konzentration ist. Es wurden Hemmeffekte zwischen 5% und 90% ermittelt. Alle Schwermetallsalze ließen sich mit den eingesetzten Konzentrationen in HEPES-Puffer lösen. Für sämtliche Metalle wurden Dosis-Wirkungsbeziehungen (Hemmkurven) aufgenommen und durch Extrapolation die toxikologisch relevanten EC-Werte erhalten. Diese geben die Konzentration der getesteten Substanz an, bei der die ONP-Glykosid-Spaltung um einen bestimmten Prozentsatz gehemmt wird, wenn als Bezugsgröße Standardwerte dienen, welche destilliertes Wasser anstelle der Testlösung enthielten.

Um ausschließen zu können, daß die Metalllösung selbst bei 405 nm absorbiert, wurden entsprechende Kontrollansätze gemessen, bei denen die Zellsuspensionen durch Puffer ersetzt wurden. Da die Schwermetallionen ausschließlich als Chloride zum Einsatz kamen wurden zusätzlich Natriumchloridlösungen als Negativkontrolle eingesetzt, um eine eventuelle Hemmung aufgrund der Anionen ausschließen zu können.

In Tabelle 4 sind die $EC_{10}-$ und $EC_{50}-$ Werte für die eingesetzten Schwermetallionen wiedergegeben, jeweils mit den in der Ökotoxikologie üblichen Konzentrationsangaben ppm und den in der Biochemie obligaten SI-Einheiten $\mu$M.

## Tabelle 4

| Metallion | EC$_{10}$ | | EC$_{50}$ | |
|---|---|---|---|---|
| | ppm | µM | ppm | µM |
| Cd$^{2+}$ | 10 | 89,0 | 19 | 169,1 |
| Cr$^{3+}$ | 8 | 153,6 | 16 | 307,2 |
| Cu$^{2+}$ | 5,5 | 86,4 | 11 | 172,7 |
| Hg$^{2+}$ | 1,3 | 6,4 | 2,1 | 10,3 |
| Ni$^{2+}$ | 0,5 | 8,5 | 4,5 | 76,6 |
| Pb$^{2+}$ | 25 | 120,0 | 67 | 321,6 |
| Zn$^{2+}$ | | | 8 | 122,4 |

Aus Tabelle 4 ist zu entnehmen, daß die Hydrolyse der ONP-Glucose auf das Vorhandensein von Ni$^{2+}$ und Hg$^{2+}$ am empfindlichsten reagiert. Bereits 0,5 ppm Ni$^{2+}$ (entsprechend 8,5 µM) und 1,3 ppm Hg$^{2+}$ - (entsprechend 6,4 µM) rufen eine 10%-ige Hemmung hervor. Der EC$_{50}$-Wert von Ni$^{2+}$ ist mit 4,5 ppm (entsprechend 76,6 µM) höher als der entsprechende EC$_{50}$-Wert von Hg$^{2+}$ mit 2,1 ppm (entsprechend 10,3 µM). Mit beiden Metallionen wurde eine maximale Hemmung von ca. 70% erzielt.

Mit Ausnahme von Pb$^{2+}$, welches mit einem EC$_{50}$-Wert von 67 ppm (entsprechend 321,6 µM) den schwächsten Hemmeffekt auf die eingesetzte E.coli-Mutante ausübte, liegen die resultierenden EC$_{10}$- und EC$_{50}$-Werte von Cu$^{2+}$, Cr$^{3+}$ und Cd$^{2+}$ mit 5,5 ppm bis 10 ppm (EC$_{10}$) bzw. 11 ppm bis 19 ppm (EC$_{50}$) vergleichsweise eng beieinander. Anders liegen die Verhältnisse jedoch bei Angabe von Stoffmengehkonzentrationen (µM): Cr$^{3+}$ hemmt erst bei einer höheren molaren Konzentration (153,6 µM) als Pb$^{2+}$ (120 µM), obwohl der entsprechende EC$_{10}$-Wert von 25 ppm bei Pb$^{2+}$ mehr als dreimal höher liegt als bei Cr$^{3+}$ mit 8 ppm.

Beispiel 2:

PTS-Hemmtest mit organischen Lösungsmitteln

Als Testsubstanzen wurden die wasserunlöslichen Lösungsmittel Chloroform, Phenol und Toluol sowie die wasserlöslichen Dioxan und Ethanol eingesetzt. Da wasserunlösliche Lösungsmittel getestet werden mußten, wurde eine mit diesen Lösungsmitteln gesättigte wässrige Phase eingesetzt. Hierfür wurden die wasserunlöslichen Lösungsmittel zunächst in destilliertes Wasser gegeben und unter ständigem Rühren über Nacht die wässrige Phase mit dem jeweiligen Lösungsmittel gesättigt. Die Konzentrationen der wässrigen Phasen an wasserunlöslichem Lösungsmittel betrugen bei Chloroform 0,81% (v/v), bei Phenol 8,2 bis 9,2% (v/v) und bei Toluol 0,047% (v/v). Diese mit Lösungsmitteln gesättigten wässrigen Phasen wurden dann in verschiedenen Verdünnungsstufen den Zellsuspensionen zugesetzt.

Als Puffersystem diente ein 50 mM Phosphatpuffer bei pH 6,5, da sich hierdurch bei der Hemmung der ONP-Glucose-Spaltung eine Steigerung der Empfindlichkeit um bis zu 20% gegenüber den anderen Puffersystemen erzielen ließ. Die anderen Parameter Inkubationszeit, Reaktionszeit, Bakterienmenge, Temperatur und Substratkonzentration wurden analog den Werten aus Beispiel 1 eingestellt.

Die toxikologisch relevanten EC-Werte wurden wie in Beispiel 1 aus Dosis-Wirkungsbeziehungen (Hemmkurven) mittels Extrapolation (Gamma-Verfahren) erhalten. In Abbildung 2 sind wieder die Hemmgeraden in doppeltlogarithmischer Darstellung wiedergegeben. Die resultierenden EC$_{10}$- bzw. EC$_{50}$ Werte wurden in Tabelle 5 angegeben.

Tabelle 5

| Lösungmittel | $EC_{10}$ | | $EC_{50}$ | |
|---|---|---|---|---|
| | ppm | mM | ppm | mM |
| Chloroform | 720 | 6,0 | 1900 | 15,9 |
| Phenol | 350 | 3,7 | 1400 | 14,9 |
| Toluol | 15 | 0,16 | 35 | 0,38 |
| Dioxan | 10000 | 114 | 30000 | 341 |
| Ethanol | 10500 | 288 | 33000 | 717 |

Wie aus der Tabelle ersichtlich ist, üben die meisten der eingesetzten organischen Lösungsmittel erst in hohen Konzentrationen hemmende Effekte auf den ONP-Glucose-Umsatz von E.coli aus. Bei den wasserunlöslichen Lösungsmitteln führte lediglich Toluol bereits in geringen Mengen zu deutlichen Hemmungen. Der $EC_{10}$-Wert wurde zu 15 ppm (entsprechend 0,16 mM) ermittelt und lag damit beinahe um den Faktor 50 unter dem entsprechenden Wert für Chloroform, welches erst bei 720 ppm (ca. 6 mM) eine ONP-Glucose-Umsatzabnahme von 10% bewirkte. Zusätzlich wurde auch versucht, die Wirkung von Dichlorethan auf den ONP-Glucose-Umsatz zu bestimmen, wobei aber keine Extrapolation zu den erforderlichen Gamma-Werten möglich war. Dichlorethan wurde dabei bis zu einer Konzentration von 3500 ppm eingesetzt. Dies entspricht einer Stoffmengenkonzentration von etwa 35 mM, wobei lediglich eine Hemmung von etwa 30% erzielt werden konnte.

Die wasserlöslichen Lösungsmittel Dioxan und Ethanol übten geringe Effekte auf die ONP-Glucose-Hydrolyse aus. Die $EC_{10}$-Werte betrugen ca. 10 000 ppm für Dioxan and etwa 10 500 ppm für Ethanol, was einer Stoffmengenkonzentration von 0,11 mol/l bzw. 0,28 mol/l entspricht.

Beispiel 3:

PTS-Hemmtest mit Detergenzien

Als Detergenzien wurden das ionische Detergenz SDS und das nicht-ionische Triton X-100 in ihrer Wirkung auf das PTS-System von E.coli 1219 bgl+ untersucht. Als Puffersystem diente ein 50 mM HEPES-Puffer bei pH 6,5. Die restlichen Reaktionsparameter entsprachen wieder den Werten nach Beispiel 1.

Die Extrapolation der Umsatzraten ergab nach Inkubation mit SDS einen $EC_{10}$-Wert von 5,5 ppm, entsprechend einer Stoffmengenkonzentration von ca. 19 $\mu$M, sowie einen $EC_{50}$-Wert von 13 ppm, entsprechend 45,1 $\mu$M. Das nichtionische Triton X-100 zeigte erst in höheren Konzentrationen eine signifikante Hemmung, entsprechend einem $EC_{10}$-Wert von ca. 70 ppm (entsprechend 108 $\mu$M) und einem $EC_{50}$-Wert von ca. 180 ppm (entsprechend 280 $\mu$M). Die Werte sind in Tabelle 6 nochmals zusammengefaßt.

Tabelle 6

| Detergens | $EC_{10}$ | | $EC_{50}$ | |
|---|---|---|---|---|
| | ppm | $\mu$M | ppm | $\mu$M |
| SDS | 5,5 | 19,1 | 13 | 45,1 |
| Triton X-100 | 70 | 108 | 180 | 278 |

Zur Verdeutlichung der Leistungsfähigkeit des erfindungsgemäßen PTS-Hemmtests werden in der folgenden Tabelle 7, die in den Beispielen ermittelten Daten entsprechend den mit dem Biolumineszenz-Hemmtest gemäß DIN 384 12, Teil 34 ermittelten Daten gegenübergestellt. Die für den Biolumineszenz-Hemmtest verwendeten Daten wurden aus der entsprechenden Literatur entnommen.

Tabelle 7

| Testsubstanz | PTS- Hemmtest $EC_{50}$ (ppm) | Reaktionszeit in min | Bioluminineszenz- Hemmtest $EC_{50}$ (ppm) | Reaktionszeit in min | Referenz * |
|---|---|---|---|---|---|
| $Cd^{2+}$ | 19 | 5 | 131 | 10 | Beckmann, 1980 |
| $Cu^{2+}$ | 11 | 5 | 16 | 10 | Beckmann, 1980 |
| $Zn^{2+}$ | 8 | 5 | 25,4 | 10 | Beckmann, 1980 |
| $Hg^{2+}$ | 2,1 | 5 | 0,065 | 5 | Bulich et al., 1981 |
| $Pb^{2+}$ | 67 | 5 | 0,4 | 30 | Beckmann, 1981 |
| Chloroform | 1900 | 5 | 1168 | 10 | Beckmann, 1980 |
| Phenol | 1400 | 5 | 25 | 5 | Bulich et al., 1981 |
| Ethanol | 33000 | 5 | 31000 | 5 | Bulich et al., 1981 |

*Beckmann, 1980; The microtox system, an approach to acute water toxicity monitoring. - Microtox slide presentation; Beckmann Instruments Inc.; Microbic Operations, Carlsbad, Calif., USA. Bulich, A.A., Greene, M.W. and Isenberg, D.L., 1981; Reliability of the bacterial luminescence assay for determination of the toxicity of pure compounds and complex effluents. In: Branson, D.R. and Dickson, K.L. (eds.): Aquatic toxicology and hazard assesment: fourth conference. ASTM STP 737: 338-347.

Im Vergleich der entsprechenden Hemmdaten bei den Schwermetallionen $Cd^{2+}$, $Cu^{2+}$ und $Zn^{2+}$ läßt sich erkennen, daß der erfindungsgemäße PTS-Test wesentlich empfindlicher reagiert als der Biolumineszenz-Hemmtest, insbesondere wenn man auch berücksichtigt, daß die Reaktionszeit von 5 Minuten nur etwa die Hälfte der Reaktionszeit des Biolumineszenz-Hemmtests betrug. Besonders deutlich ist das an den jeweiligen EC-Werten, die nach Inkubation mit $Cd^{2+}$ ermittelt wurden zu erkennen. Im PTS-Hemmtest wurde eine 50%-ige Hemmung bereits durch 19 ppm $Cd^{2+}$ erreicht. Wenn man berücksichtigt, daß der Substratumsatz proportional zur Reaktionszeit ist, dann ist der erfindungsgemäße Test um den Faktor 14 empfindlicher als der Biolumineszenz-Hemmtest, bei dem erst bei einer Konzentration von 131 ppm nach einer Reaktionszeit von 10 Minuten die entsprechende Inhibierung vezeichnet werden konnte. Darüber hinaus bildet der erfindungsgemäße Test für $Cr^{3+}$ mit einem $EC_{50}$-Wert von 16 ppm einen empfindlichen Hemmtest. Entsprechende Daten mit dem Biolumineszenz-Hemmtest konnten in der Literatur nicht gefünden werden.

Deutlich unempfindlicher als im Biolumineszenz-Hemmtest wirkt sich dagegen die Anwesenheit von $Pb^{2+}$ und $Hg^{2+}$ auf den ONP-Glukose-Umsatz aus, während für die organischen Lösungsmittel Chloroform und Ethanol die erzielten Empfindlichkeiten in etwa in der gleichen Größenordnung liegen. Für Phenol erweist sich jedoch der Biolumineszenz-Hemmtest als deutlich empfindlicher.

Ein Vergleich der EC-Werte in Bezug auf Detergenzien mit dem Biolumineszenz-Test war mangels Literaturdaten nicht möglich. Sowohl das kationische SDS als auch nicht-ionisches Triton X-100 zeigten im erfindungsgemäßen PTS-Hemmtest bei relativ niedrigen Konzentrationen signifikante Hemmungen. Die $EC_{10}$-Werte wurden für SDS zu 5,5 ppm und für Triton X-100 zu 70 ppm ermittelt und lagen damit in der Größenordnung der Hemmraten für Schwermetalle.

Der erfindungsgemäße PTS-Test stellt in Bezug auf seine Handhabung ein einfach durchzuführendes Testsystem dar. Durch die Möglichkeit, eingefrorene Bakterien einzusetzen, ist der Test ohne Vorbereitung durchführbar. Mit der zuvor beschriebenen Konservierungsmethode für die einzusetzende Bakterierkulturen lassen sich im voraus Testorganismen für beliebig viele Tests bereitstellen. Da der eigentliche Hemmtest von der Kultivierung der Mikroorganismen getrennt ist, benötigt man zur Durchführung keinen sterilen Arbeitsplatz und der Test kann mit Labor-Standardgeräten (Zentrifuge, Photometer, temperierbares Wasserbad) durchgeführt werden. Somit stellt das erfindungsgemäße Verfahren ein äußerst wünschenswerten Test, insbesondere infolge seiner hohen Empfindlichkeit gegenüber den Schwermetallionen $Cd^{2+}$, $Cu^{2+}$, $Zn^{2+}$ und insbesondere auch $Cr^{3+}$ zur Verfügung und schließt damit eine Lücke zu den im Stand der Technik bekannten Tests zum Nachweis von Schadstoffen.

**Patentansprüche**

1. Verfahren zum Nachweis von Schadstoffen mittels Bestimmung der Wirkung der in einer Probe enthaltenen Schadstoffe auf den Stoffwechsel von Mikroorganismen, dadurch gekennzeichnet, daß als Nachweis für die Gegenwart von Schadstoffen in einer Probe deren Hemmwirkung auf das Phosphotransferase-System von Bakterien dient.

2. Verfahren nach Anspruch 1, dadurch gekennzeichnet, daß es die Schritte umfaßt: Herstellen einer Suspension von über das Phosphotransferase-System verfügenden Bakterien zusammen mit der zu untersuchenden Probe in einem Puffersystem und Zugabe eines Substratanalogen für ein mittels des Phosphoenolpyruvat-abhängigen Phosphotransferase-Systems unter Phosphorylierung in die Bakterienzelle transportierten Kohlenhydrats, wobei dieses Substratanaloge so beschaffen ist, daß es nach seiner Phosphorylierung von einem bakterieneigenen Enzym des Phosphotransferase-Systems hydrolisiert werden kann unter Freisetzung einer analytisch erfaßbaren Substanz, deren Konzentration sodann nach einer definierten Reaktionszeit nach Zugabe des Substratanalogen quantitativ bestimmt wird und der erhaltene Wert mit einem aus einem Vergleichsansatz einer Suspension ohne Probezusatz erhaltenen Wert verglichen wird.

3. Verfahren nach den Ansprüchen 1 und 2, dadurch gekennzeichnet, daß die aus dem Substratanalogen freigesetzte analytisch erfaßbare Substanz photometrisch bestimmt wird.

4. Verfahren nach den Ansprüchen 1-3, dadurch gekennzeichnet, daß das Substratanaloge ein 2-Nitrophenyl-glycosid ist.

5. Verfahren nach jedem der Ansprüche 1-4, dadurch gekennzeichnet, daß als Bakterium eine Mutante von E.coli mit der Bezeichnung 1219 bgl+ und der Hinterlegungsnummer DSM 8779 eingesetzt wird.

6. Verfahren nach den Ansprüchen 4 und 5, dadurch gekennzeichnet, daß das 2-Nitrophenyl-Glycosid 2-Nitrophenyl-$\beta$-D-Glucosid ist.

7. Verfahren nach den Ansprüchen 1-6, dadurch gekennzeichnet, daß Bakterienkulturen eingesetzt werden, welche auf - 20° C eingefroren waren und vor der Zugabe zur Suspension aufgetaut worden sind.

8. Verfahren nach Anspruch 7, dadurch gekennzeichnet, daß die Bakterien vor dem Einfrieren mit einer 10%-igen Rinderserumalbuminlösung gewaschen werden.

9. Verfahren nach Anspruch 7, dadurch gekennzeichnet, daß die eingefroreren Bakterien zunächst über einen Zeitraum von 10 Minuten bei Raumtemperatur aufgetaut und sodann über einen Zeitraum von 10-15 Minuten bei 37° C in einem für physiologische Zwecke geeigneten Puffersystem mit einem pH zwischen 6 und 8,5 vorinkubiert werden.

10. Verfahren nach den Ansprüchen 1-3, dadurch gekennzeichnet, daß als Puffersystem ein 50 mM HEPES-Puffer mit einem pH von 6,5 verwendet wird.

11. Verfahren nach den Ansprüchen 1-3, dadurch gekennzeichnet, daß als Puffersystem ein 50 mM Phosphat-Puffer mit einem pH von 6,5 verwendet wird.

12. Verfahren nach den Ansprüchen 1, 2 und 5, dadurch gekennzeichnet, daß pro Testansatz die Bakterien Mutante E.coli 1219 in einer solchen Menge eingesetzt wird, daß die $OD_{578}$ der Bakteriensuspension 3,0 beträgt.

13. Verfahren nach den Ansprüchen 1-6, dadurch gekennzeichnet, daß nach Zugabe des Substratanalogen zur Mikroorganismensuspension mit der jeweiligen Probe die Suspension zur Hydrolyse des 2-Nitrophenyl-$\beta$-D-Glucosid oder des 4-Nitrophenyl-$\beta$-D-Glucosids über einen Zeitraum von 5 Minuten bei 37° C inkubiert wird.

14. Verfahren nach den Ansprüchen 3-6 und 13, dadurch gekennzeichnet, daß nach Ende der Inkubation die Hydrolyse des 2-Nitrophenyl-$\beta$-D-Glucosid oder des 4-Nitrophenyl-$\beta$-D-Glucosids durch Zugabe von 0,5 M $Na_2CO_3$-Lösung beendet wird.

15. Verfahren nach den Ansprüchen 1-6 und 13-14, dadurch gekennzeichnet, daß nach Beendigung der Hydrolyse die Zellen während 2 Minuten mit 17 000 g abzentrifügiert werden und danach die Konzentration des 2-Nitrophenolat-Anions oder der 4-Nitrophenolat-Anions im Überstand photometrisch bei 405 nm bestimmt wird.

16. E.coli Mutante 1219 bgl + mit der Hinterlegungsnummer DSM 8779.

17. Eingefrorene Zellsuspension der E.coli Mutante gemäß Anspruch 15.

## Figur 1

Restaktivität in %

BSA-Konzentration in % (w/v)

## Figur 2

nmol ONP⁻/ml

pH

**Figur 3**

**Figur 4**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | EP-A-0 286 039 (THE FLENDERS UNIVERSITY OF SOUTH AUSTRALIA) 12.Oktober 1988 *siehe das gesamte Dokument* --- | 1-22 | G01N33/20 G01N33/52 G01N33/68 C12N1/00 |
| Y | BIOCHEMICA BIOPHYSICA ACTA, Bd. 786, 1984 Seiten 231-244, G. TESORIERE ET AL. 'The purification and properties of nucleoside phosphotransferase from mucosa of chicken intestine' *sihe das gesamte Dokument* --- | 1-22 | |
| Y | BIOCHEMICA BIOPHYSICA ACTA, Bd. 481, 1977 Seiten 105-114, V.L. CROW ET AL. 'The effect of monovalent and divalent cations on the activity of S. lactis C10 pyruvate kinase' *siehe das gesamte Dokument* --- | 1-22 | |
| Y | J. ENZYME INHIBITION, Bd. 1, 1986 Seiten 113-125, L. CISKANIK ET AL. 'Inhibition and inactivation of pyruvate phosphate dikinase...' *siehe das gesamte Dokument* --- | 1-22 | **RECHERCHIERTE SACHGEBIETE (Int.Cl.6)** G01N C12N |
| Y | JOURNAL OF BIOLOGICAL CHEMISTRY, Bd. 261, no.13, 1986 Seiten 5892-5900, I.A. BRAND ET AL. 'Zn-dependent reversible inactivation of rat liver phosphofructokinase-1' *sihe das gesamte Dokument* --- -/-- | 1-22 | |

Der vorliegende Recherchenbericht wurde für alle Patentansprüche erstellt

| Recherchenort | Abschlußdatum der Recherche | Prüfer |
|---|---|---|
| MÜNCHEN | 6.April 1995 | Marie, A |

EPO FORM 1503 03.82 (P04C03)

Europäisches
Patentamt

**EUROPÄISCHER RECHERCHENBERICHT**

Nummer der Anmeldung

**EP 95 10 0589**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | ARCHIVES OF BIOCHEMISTRY AND BIOPHYSICS, Bd. 204, no.1, 1980 Seiten 288-293, K. WAKU ET AL. 'The effects of cadmium ions and cadmium-metallothionein on the activities of phospholipid-synthesizing enzymes of rat liver microsomes in vitro' *siehe das gesamte Dokument* ----- | 1-22 | |
| | | | **RECHERCHIERTE SACHGEBIETE (Int.Cl.6)** |

Der vorliegende Recherchenbericht wurde für alle Patentansprüche erstellt

| Recherchenort | Abschlußdatum der Recherche | Prüfer |
|---|---|---|
| MÜNCHEN | 6.April 1995 | Marie, A |

EPO FORM 1503 03.82 (P04C03)